(19) Europäisches Patentamt
European Patent Office
Office européen des brevets

(11) Veröffentlichungsnummer: **0 370 955 B1**

(12) **EUROPÄISCHE PATENTSCHRIFT**

(45) Veröffentlichungstag der Patentschrift: **12.10.94**

(51) Int. Cl.[5]: **C07D 231/56**, A01N 43/56, C07D 487/04, C07D 471/04, C07D 401/12, C07D 403/12, C07D 405/12, C07D 409/12, C07D 417/12, A01N 43/653, A01N 43/58

(21) Anmeldenummer: **89810875.8**

(22) Anmeldetag: **16.11.89**

Die Akte enthält technische Angaben, die nach dem Eingang der Anmeldung eingereicht wurden und die nicht in dieser Patentschrift enthalten sind.

(54) **Herbizid wirksame N-Phenylazole.**

(30) Priorität: **25.11.88 CH 4384/88**

(43) Veröffentlichungstag der Anmeldung: **30.05.90 Patentblatt 90/22**

(45) Bekanntmachung des Hinweises auf die Patenterteilung: **12.10.94 Patentblatt 94/41**

(84) Benannte Vertragsstaaten: **CH DE FR GB LI**

(56) Entgegenhaltungen:
**EP-A- 0 104 484**
**EP-A- 0 104 532**
**EP-A- 0 138 527**

(73) Patentinhaber: **CIBA-GEIGY AG**
**Klybeckstrasse 141**
**CH-4002 Basel (CH)**

(72) Erfinder: **Pissiotas, Georg, Dr.**
**Am Sonnenrain 71**
**D-7850 Lörrach (DE)**
Erfinder: **Moser, Hans, Dr.**
**Hauptstrasse 67B**
**CH-4312 Magden (CH)**
Erfinder: **Brunner, Hans-Georg, Dr.**
**Wannenstrasse 14**
**CH-4415 Lausen (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 370 955 B1

## Beschreibung

Die Erfindung betrifft neue jeweils in 2-Stellung phenylsubstituierte Pyrazole, 4,5,6,7-Tetrahydro-indazole und 2,4,5,6-Tetrahydro-cyclopenta[c]-pyrazole mit herbizider Wirkung, agrochemische Mittel, die diese Verbindungen enthalten, deren Verwendung zur Bekämpfung unerwünschten Pflanzenwachstums sowie Verfahren zur Herstellung der erfindungsgemässen Verbindungen.

Derartige Verbindungen werden beispielsweise in EP-A-0 138 527, EP-A-0 104 484 und EP-A-0 104 532 ebenfalls beschrieben. Sie enthalten aber entweder unterschiedliche Estergruppen oder unterschiedliche heterocyclische Ringsysteme am Benzolkern.

Die neuen Verbindungen entsprechen der Formel I

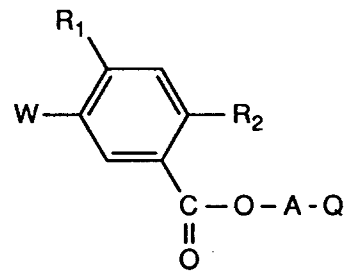

(I),

worin

W

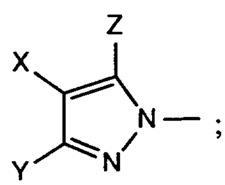

| | |
|---|---|
| $R_1$ | Wasserstoff; oder Halogen; |
| $R_2$ | Halogen; |
| A | eine geradkettige oder verzweigte $C_1$-$C_4$-Alkylenkette; |
| n | 0; oder 1; |
| Q | -$S(O)_m$-$R^3$; Di-$C_1$-$C_4$-Alkylamino; einen über Kohlenstoff gebundenen 6-gliedrigen Heterocyclus, welcher 1 bis 3 Stickstoffatome enthält, wobei dieser Heterocyclus seinerseits noch bis zu zweifach durch Halogen, $C_1$-$C_4$-Alkyl oder $C_1$-$C_3$-Halogenalkyl substituiert sein kann; |
| $R^3$ | $C_1$-$C_{10}$-Alkyl; |
| m | 0; 1; oder 2; |
| X | Wasserstoff; Halogen; oder $C_1$-$C_4$-Alkyl; |
| Y | $C_1$-$C_4$-Alkyl; oder |
| X und Y | gemeinsam eine gegebenenfalls bis zu zweifach durch Methyl substituierte -$(CH_2)_q$-Kette; |
| q | 3; oder 4; und |
| Z | Halogen; Methyl; $C_1$-$C_6$-Alkoxy; oder $C_1$-$C_6$-Alkylthio; |

bedeutet.

Die jeweils durch Semikolon voneinander abgetrennten Einzelbedeutungen der Elemente W, $R^1$ bis $R^4$, A, n, Q, m, p, X, Y und Z sind als Untergruppen dieser Substituenten anzusehen. Die Erfindung umfasst auch die durch Streichung einer oder mehrerer dieser Untergruppen erhältlichen Definitionen der Verbindungen der Formel I im jeweiligen Umfang des allgemeinsten und der bevorzugten oder besonders bevorzugten Bereiche.

In den in dieser Beschreibung verwendeten Definitionen umfassen die angegebenen generischen Begriffe, sowie die durch Kombination einzelner Unterbegriffe erhältlichen Substituenten, beispielsweise die folgenden spezifischen Einzelsubstituenten, wobei diese Aufzählung keine Beschränkung der Erfindung darstellt:

Halogen: Fluor, Chlor, Brom und Jod vorzugsweise Fluor, Chlor und Brom. Bei $R^1$ bevorzugt Fluor und

Chlor und bei $R^2$ bevorzugt Chlor und Brom.

A als $C_1$-$C_4$-Alkylenkette ist Methano, Ethano (Ethan-1,2-diyl), 1-Methylethano, 2-Methylethanol, Propano (Propan-1,3-diyl), 1-Methylpropano, 2-Methylpropano, 3-Methylpropano, Butano und Ethan-1,1-diyl. Bevorzugt sind neben Ethano die in Nachbarstellung zum Sauerstoff verzweigten Alkylenketten, wie 1-Methylethano oder 1-Ethylethano, insbesondere aber Ethano und 1-Methylethano.

Q als $-S(O)_m-R^3$ ist ein Thioether (m = 0), ein $R^3$-Sulfinyl-Radikal (m = 1) oder ein $R^3$-Sulfonyl-Radikal (m = 2).

In den Fällen, in denen $R^3$ Alkyl bedeutet sind die Thioether bevorzugt.

Di-Alkylamino ist z.B. Dimethylamino, Methylethylamino, Diethylamino, Di-Butylamin und Di-Isopropylamino.

Q als Heterocyclus ist insbesondere Pyridin-2-yl.

Alkyl ist z.B. Methyl, Ethyl, Isopropyl, n-Propyl, n-Butyl, iso-Butyl, sek-Butyl, tert-Butyl sowie die verschiedenen isomeren Pentyl-, Hexyl-, Heptyl-, Octyl-, Nonyl- und Decylradikale.

Halogenalkyl ist z.B. Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Trichlormethyl, Difluorchlormethyl, Trifluormethyl und Dichlorfluormethyl.

Alkoxy ist z.B. Methoxy, Ethoxy, Propyloxy, i-Propyloxy, n-Butyloxy, i-Butyloxy, s-Butyloxy und t-Butyloxy; vorzugsweise Methoxy.

Halogenalkoxy ist z.B. Fluormethoxy, Difluormethoxy, Trifluormethoxy, 2,2,2-Trifluorethoxy, 1,1,2,2-Tetrafluroethoxy, 2-Fluorethoxy, 2-Chlorethoxy und 2,2,2-Trichlorethoxy; vorzugsweise Difluormethoxy, 2-Chlorethoxy und Trifluormethoxy.

Alkylthio ist z.B. Methylthio, Ethylthio, Propylthio, Isopropylthio, n-Butylthio, i-Butylthio, s-Butylthio oder t-Butylthio; vorzugsweise Methylthio und Ethylthio.

Der Substituent W kann in Formel I für verschiedene heterocyclische Ringsysteme stehen. Im einzelnen sind die folgenden Gruppen von Verbindungen gemeint:

Pyrazole der Formel Ia

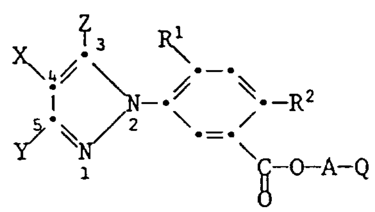

(Ia),

2,4,5,6-Tetrahydro-cyclopenta[c]pyrazole der Formel Ib

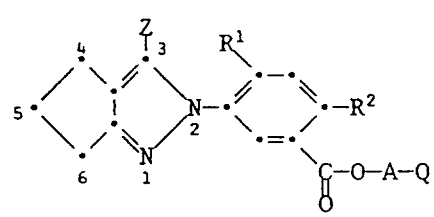

(Ib)

und

4,5,6,7-Tetrahydro-indazole der Formel Ic

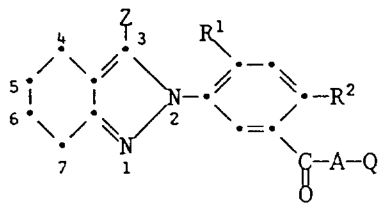

(Ic) ,

Bevorzugt sind Verbindungen der Formel I,
worin

W wie zuvor definiert ist und
$R^1$ Wasserstoff; Fluor; oder Chlor;
$R^2$ Fluor; Chlor; oder Brom;
A eine geradkettige oder verzweigte $C_2$-$C_4$-Alkylenkette;
n 0; oder 1;
Q $C_1$-$C_{10}$-Alkylthio; Di-$C_1$-$C_4$-Alkylamino; gegebenenfalls bis zu zweifach durch $C_1$-$C_4$-Alkyl substituiertes Pyridinyl;
X Wasserstoff; Chlor; oder Brom;
Y $C_1$-$C_4$-Alkyl; oder
X und Y gemeinsam eine $-(CH_2)_q$-Kette;
q 3; oder 4; und
Z Chlor; Brom; Methyl; $C_1$-$C_6$-Alkoxy; oder $C_1$-$C_6$-Alkylthio;

bedeutet.

Besonders bevorzugt sind Verbindungen der Formel I,
worin

W wie zuvor definiert ist und
$R^1$ Wasserstoff; Fluor; oder Chlor;
$R^2$ Chlor; oder Brom;
A Ethano; 1-Methylethano; 2-Methylethano; oder Propano;
n 0; oder 1;
Q $C_1$-$C_5$-Alkylthio; oder Pyridin-2-yl;
X und Y gemeinsam eine $-(CH_2)_4$- Kette; und
Z Chlor; oder Methyl;

bedeutet.

Besonders hervorzuheben sind diejenigen Verbindungen der Formel I, worin

a)
W

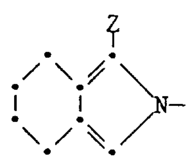

und
Z Chlor; oder Methyl;
bedeutet;

f) A $-CH_2$-$CH_2$- bedeutet;

g)

A 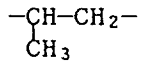

bedeutet;

h)

A 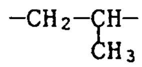

bedeutet;

i) Q $C_1$-$C_6$-Alkylthio bedeutet;

j) Q Di-$C_1$-$C_4$-Alkylamino bedeutet;

k) $R^1$ Fluor; und
$R^2$ Chlor; oder Brom; bedeutet;

l) $R^1$ Fluor; und
$R^2$ Chlor; bedeutet.

Im Hinblick auf die gute Herbizidwirkung sind insbesondere folgende Kombinationen der vorgenannten Einzelmerkmale hervorzuheben.

m) Die Kombination der Merkmale a) f), g), und i)

Besonders gut wirksam sind Verbindungen der Formel I, worin

W

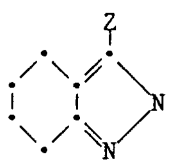

Z Chlor; oder Methyl;

A $-CH_2-CH_2-$; oder

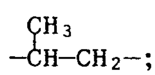

Q $C_1$-$C_6$-Alkylthio;

$R^1$ Fluor; und

$R^2$ Chlor; oder Brom;

bedeutet.

Namentlich zu nennen sind insbesondere

3-Chlor-2-{4-chlor-2-fluor-5-[1-methyl-2-(1-methylethylthio)-ethoxycarbonyl]-phenyl}-4,5,6,7-tetrahydro-2H-indazol,

3-Chlor-2-[4-chlor-2-fluor-5-(1-dimethylamino-1-methylethoxycarbonyl)-phenyl]-4,5,6,7-tetrahydro-1H-indazol und

3-Chlor-2-[4-chlor-2-fluor-5-(2-ethylthio-1-methylethoxycarbonyl)-phenyl]-4,5,6,7-tetrahydro-2H-indazol.

Die Verbindungen der Formel I können hergestellt werden durch

a) Veresterung eines Carbonsäurederivats der Formel II, worin $R^1$, $R^2$ und W wie zuvor definiert sind und $R^5$ OH, Chlor oder Brom bedeutet, mit einem Alkohol der Formel III, worin A und Q wie zuvor definiert sind

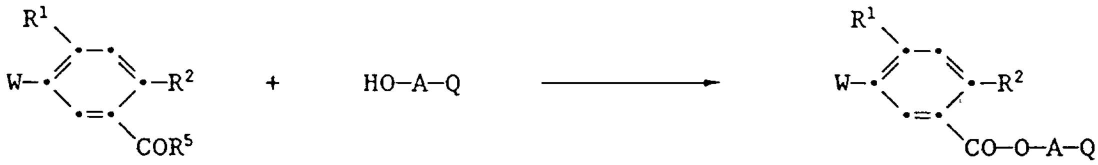

II III I .

Die Ausgangsverbindungen der Formel II und III sind bekannt oder können analog zu literaturbekannten Verfahren hergestellt werden.

Die zur Synthese der Pyrazole, Indazole bzw. Cyclopenta[c]pyrazole der Formeln Ia, Ib bzw. Ic erforderlichen Carbonsäurederivate der Formel II sind in der EP-A-0 138 527 beschrieben.
Verfahren zur Herstellung der Ausgansverbindungen der Formel II sind auch in der EP-A-0 347 382 beschrieben.

Die europäischen Patentanmeldungen EP-A-0 104 532 und EP-A-0 104 484 betreffen überwiegend einfache Ester der Formel II ($R^5$ = gegebenenfalls substituiertes Alkoxy) aus diesen Estern können durch Verseifen und gegebenenfalls anschliessendes Chlorieren oder Bromieren (etwa mit $POCl_3$ oder $POBr_3$) die entsprechenden freien Carbonsäuren ($R^5$ = OH) oder die Säurechloride bzw. -bromide ($R^5$ = Cl oder Br) der Formel II hergestellt werden.

Die erfindungsgemässe Veresterungsreaktion ist ein dem Fachmann geläufiges Verfahren.

Die Umsetzung der Säurechloride bzw. -bromide der Formel II ($R^5$ = Cl oder Br) mit den Alkoholen der Formel III wird mit Vorteil in einem reaktionsinerten Lösungsmittel bei Temperaturen 0 bis zur Siedetemperatur des Lösungsmittels, insbesondere bis etwa 120 ° C, durchgeführt.
Dabei kann es von Vorteil sein, Basen zuzusetzen, welche das freiwerdende HCl bzw. HBr binden.

Es ist auch eine direkte Veresterung der freien Carbonsäure der Formel II ($R^5$ = OH) möglich. Dann werden wasserabspaltende bzw. wasserbindende Mittel benötigt.
Wasserabspaltende Mittel sind beispielsweise Protonensauren.
Als wasserbindendes Mittel sind insbesondere Carbodiimide, wie Cyclohexylcarbodiimid, zu erwähnen.
Auch die Verfahren ausgehend von den Carbonsäuren II werden mit Vorteil in einem reaktionisierten Lösungsmittel bei Temperaturen zwischen 0 ° C und etwa 100 ° C durchgeführt.
Bei der Carbodiimid-Methode sind tiefe Temperaturen von 0 ° C bis etwa Raumtemperatur bevorzugt.

Ausserdem können die Verbindungen der Formel I", worin die Reste W, $R^1$, $R^2$, $R^3$ und A wie zuvor definiert sind und Q für $-S(O)_m-R^3$ und m für 1 oder 2 steht, hergestellt werden durch

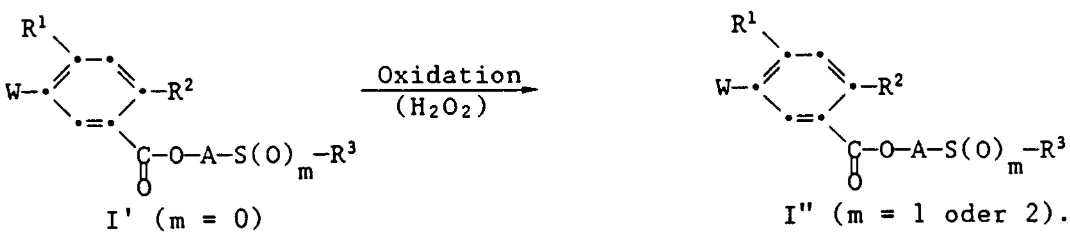


I' (m = 0) I" (m = 1 oder 2).

Oxidation von Thioethern der Formel I', worin die Reste W, $R^1$, $R^2$, $R^3$ und A wie zuvor definiert sind und m für 0 steht.

Die Oxidationen werden vorzugsweise in Lösung bei Temperaturen von 0 ° C bis 100 ° C durchgeführt.

Derartige Oxidationen sind dem Fachmann geläufig. Sie sind unter anderem beschrieben in Houben Weyl, Methoden der Organischen Chemie, Bd. IX, S. 211 ff. Geeignete Oxidationsmittel sind neben Wasserstoffperoxid, oder Persäuren, wie etwa m-Chlorperbenzoesäure und Kaliumpermanganat in Eisessig, etwa analog J. Amer. Chem. Soc. 63 [1941] 1971 oder analog Can. J. Chem. 57 (1979) 2426).

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Aufwandmengen zeichnen sich die Wirkstoffe der Formel I durch gute selektivherbizide Eigenschaft gegen Unkräuter aus. Kulturpflanzen wie Roggen, Gerste, Hafer, Weizen, Mais, Sorghum, Reis, Baumwolle und Soja bleiben bei niedrigen Aufwandmengen praktisch ungeschädigt. Bei gesteigerten Aufwandmengen werden die Kulturpflanzen nur geringfügig in ihrem Wachstum beeinflusst.

Werden sehr hohe Aufwandmengen appliziert, entfalten die Substanzen der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung verwendet werden. Insbesondere eignen sich die erfindungsgemässen Verbindungen als Selektivherbizide bei preemergenter Applikation in folgenden Kulturen: Gerste, Weizen, Mais, Reis, Sorghum, Soja, Baumwolle und Sonnenblumen, sowie bei postemergenter Applikation in folgenden Kulturen: Gerste, Weizen, Mais, Reis und Sorghum.

In vorteilhafter Weise können die erfindungsgemässen Wirkstoffe oder Mittel auch auf das Vermehrungsgut der Kulturpflanze aufgebracht werden. Besonders zu erwähnen ist hier die Samenbeizung. Vermehrungsgut sind Samen, Stecklinge oder sonstige Teile der Pflanze, aus denen die Kulturpflanze gezogen werden kann. Das mit einer wirksamen Menge einer Verbindung der Formel I behandelte Vermehrungsgut ist ebenfalls Gegenstand der Erfindung.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-laurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensations-produktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanolerwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"1986 International Mc Cutcheon's Emulsifiers & Detergents" Glen Rock, N.J., USA, 1986; H. Stache, "Tensid-Taschenbuch", 2. Auflage.,

C. Hanser Verlag, München, Wien, 1981;

M. and J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

| Emulgierbare Konzentrate: | | | |
|---|---|---|---|
| Aktiver Wirkstoff: | 1 bis 20 %, | bevorzugt | 5 bis 10 % |
| oberflächenaktive Mittel: | 5 bis 30 %, | vorzugsweise | 10 bis 20 % |
| flüssige Trägermittel: | 50 bis 94 %, | vorzugsweise | 70 bis 85 % |

| Stäube: | | | |
|---|---|---|---|
| Aktiver Wirkstoff: | 0,1 bis 10 %, | vorzugsweise | 0,1 bis 1 % |
| festes Trägermittel: | 99,9 bis 90 %, | vorzugsweise | 99,9 bis 99 % |

| Suspensions-Konzentrate: | | | |
|---|---|---|---|
| Aktiver Wirkstoff: | 5 bis 75 %, | vorzugsweise | 10 bis 50 % |
| Wasser: | 94 bis 25 %, | vorzugsweise | 88 bis 30 % |
| oberflächenaktives Mittel: | 1 bis 40 %, | vorzugsweise | 2 bis 30 % |

| Benetzbares Pulver: | | | |
|---|---|---|---|
| Aktiver Wirkstoff:<br>oberflächenaktives Mittel:<br>festes Trägermittel: | 0,5 bis 90 %,<br>0,5 bis 20 %,<br>5 bis 95 %, | vorzugsweise<br>vorzugsweise<br>vorzugsweise | 1 bis 80 %<br>1 bis 15 %<br>15 bis 90 % |

| Granulate: | | | |
|---|---|---|---|
| Aktiver Wirkstoff:<br>festes Trägermittel: | 0,5 bis 30 %,<br>99,5 bis 70 %, | vorzugsweise<br>vorzugsweise | 3 bis 15 %<br>97 bis 85 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele erläutern die Erfindung.

Beispiele:

H.1.1. Herstellung von 3-Chlor-2-{4-chlor-2-fluor-5-[1-methyl-2-(1-methylethylthio)-ethoxycarbonyl]-phenyl}-4,5,6,7-tetrahydro-2H-indazol

Zu einer Lösung von 1,4 g (1-Methylethyl)thiopropan-2-ol und 1,5 ml Triethylamin in 50 ml Toluol tropft man unter Rühren bei Raumtemperatur eine Lösung von 3,5 g 3-Chlor-2-(4-chlor-2-fluor-5-chlorcarbonylphenyl)-4,5,6,7-tetrahydro-2H-indazol in 50 ml Toluol. Nach Beendigung der Zugabe wird noch weitere 5 Stunden bei Raumtemperatur gerührt, vom Triethylamin-Hydrochlorid abfiltriert und das Filtrat im Vakuum eingeengt.

Man isoliert 3,1 g der Titelverbindung der Formel

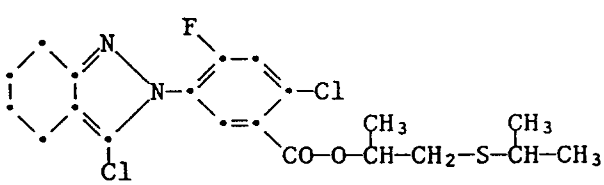

als Oel mit Brechungsindex $n_D^{24}$ = 1,5526 (Verb. No. 1.05).

Analog zu vorstehendem Beispiel und den in der Beschreibung genannten Herstellungsverfahren können die Verbindungen der Tabellen 1, 2, 7 und 8 hergestellt werden.

Tabelle 1

Verbindungen der Formel

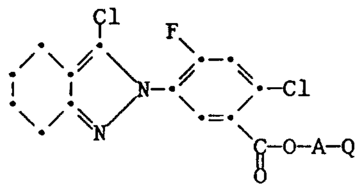

| Verb. Nr. | A | Q | physikalische Daten |
|---|---|---|---|
| 1.01 | $-CH_2-CH_2-$ | $SCH_3$ | |
| 1.02 | $-CH-CH_2-$<br>$CH_3$ | $SCH_3$ | $n_D^{21}$ 1,5655 |
| 1.03 | $-CH-CH_2-$<br>$CH_3$ | $SC_2H_5$ | $n_D^{24}$ 1,5538 |
| 1.04 | $-CH-CH_2-$<br>$CH_3$ | $SC_3H_7$ | $n_D^{24}$ 1,5339 |
| 1.05 | $-CH-CH_2-$<br>$CH_3$ | $SC_3H_7(i)$ | $n_D^{24}$ 1,5526 |
| 1.06 | $-CH-CH_2-$<br>$CH_3$ | $SC_4H_9$ | $n_D^{24}$ 1,5411 |
| 1.07 | $-CH-CH_2-$<br>$CH_3$ | $SC_4H_9(s)$ | $n_D^{24}$ 1,5349 |
| 1.08 | $-CH-CH_2-$<br>$CH_3$ | $SC_4H_9(t)$ | $n_D^{24}$ 1,5451 |
| 1.09 | $-CH-CH_2-$<br>$CH_3$ | $SC_5H_{11}$ | |
| 1.10 | $-CH-CH_2-$<br>$CH_3$ | $SC_6H_{13}$ | |
| 1.11 | $-CH-CH_2-$<br>$CH_3$ | $SC_7H_{15}$ | |
| 1.12 | $-CH-CH_2-$<br>$CH_3$ | $SC_8H_{17}$ | |
| 1.13 | $-CH-CH_2-$<br>$CH_3$ | $SC_9H_{19}$ | |
| 1.14 | $-CH-CH_2-$<br>$CH_3$ | $SC_{10}H_{21}$ | |
| 1.15 | $-CH-CH_2-$<br>$CH_3$ | $SCH_2-$ 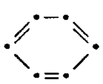 | |
| 1.16 | $-CH-CH_2-$<br>$CH_3$ | $-S-$ 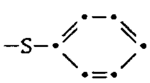 | |

Tabelle 1 (Fortsetzung)

| Verb. Nr. | A | Q | physikalische Daten |
|---|---|---|---|
| 1.17 | $-CH-CH_2-$<br>$CH_3$ | 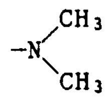 | $n_D^{24}$ 1,5603 |
| 1.18 | $-CH-CH_2-$<br>$CH_3$ | 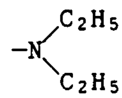 | $n_D^{25}$ 1,5458 |
| 1.19 | $-CH_2-CH_2-$ | 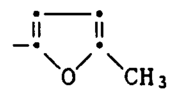 | |
| 1.20 | $-CH_2-CH_2-$ | 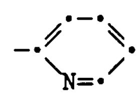 | Smp.: 75-77° |
| 1.21 | $-CH_2-CH_2-$ | 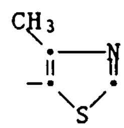 | |
| 1.22 | $-CH_2-CH_2-$ | 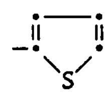 | |
| 1.23 | $-CH_2-CH_2-$ | 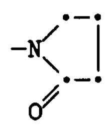 | |
| 1.24 | $-CH_2-CH_2-$ | 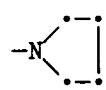 | |
| 1.25 | $-CH_2-CH_2-$ | 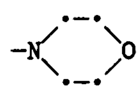 | |
| 1.26 | $-CH-CH_2-$<br>$CH_3$ | 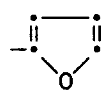 | |
| 1.27 | $-CH_2-CH_2-$ | 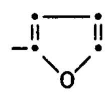 | |
| 1.28 | $-CH-CH_2-$<br>$CH_3$ | 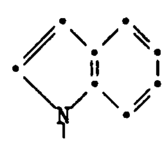 | |

Tabelle 1 (Fortsetzung)

| Verb. Nr. | A | Q | physikalische Daten |
|---|---|---|---|
| 1.29 | $-CH_2-CH_2-$ | 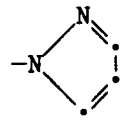 | |
| 1.30 | $-CH_2-CH_2-$ | 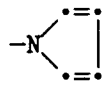 | |
| 1.31 | $-CH_2-CH_2-$ | 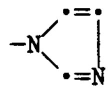 | |
| 1.32 | $CH_3$<br>$-CH-CH_2-$ | 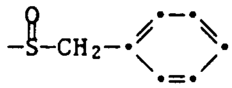 | |
| 1.33 | $CH_3$<br>$-CH-CH_2-$ | 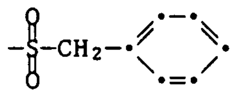 | |
| 1.34 | $CH_3$<br>$-CH-CH_2-$ | 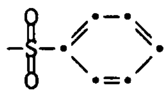 | |
| 1.35 | $-CH_2-CH_2-$ | 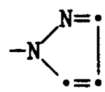 | |
| 1.36 | $CH_3$<br>$-CH-CH_2-$ | 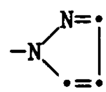 | |
| 1.37 | $CH_3$<br>$-CH-CH_2-$ | 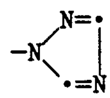 | |
| 1.38 | $CH_3$<br>$-CH-CH_2-$ | 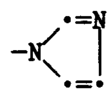 | |
| 1.39 | $-CH-CH_2-$<br>$CH_3$ | 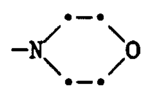 | |

Tabelle 2

Verbindungen der Formel

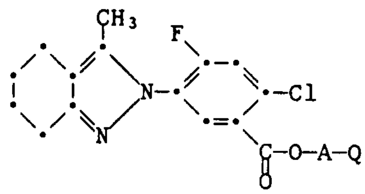

| Verb. Nr. | A | Q | physikalische Daten |
|---|---|---|---|
| 2.01 | $-CH_2CH_2-$ | $-SCH_3$ | |
| 2.02 | $CH_3$<br>$-CHCH_2-$ | $-SCH_3$ | |
| 2.03 | $CH_3$<br>$-CHCH_2-$ | $-SC_2H_5$ | |
| 2.04 | $CH_3$<br>$-CHCH_2-$ | $-SC_3H_7(i)$ | |
| 2.05 | $CH_3$<br>$-CHCH_2-$ | $-SC_4H_9$ | |
| 2.06 | $CH_3$<br>$-CHCH_2-$ | $-SC_4H_9(s)$ | |
| 2.07 | $CH_3$<br>$-CHCH_2-$ | $-SC_4H_9(t)$ | |
| 2.08 | $CH_3$<br>$-CHCH_2-$ | $-SC_8H_{17}$ | |
| 2.09 | $CH_3$<br>$-CHCH_2-$ | $-SCH_2-$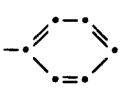 | |
| 2.10 | $CH_3$<br>$-CHCH_2-$ | 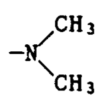 | |
| 2.11 | $-CH_2CH_2-$ | 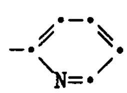 | |
| 2.12 | $-CH_2CH_2-$ | 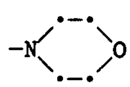 | |
| 2.13 | $-CH_2CH_2-$ | 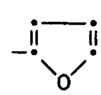 | |
| 2.14 | $CH_3$<br>$-CHCH_2-$ | 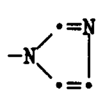 | |

Tabelle 2 (Fortsetzung)

| Verb. Nr. | A | Q | physikalische Daten |
|---|---|---|---|
| 2.15 | 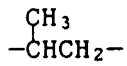 | 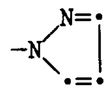 | |
| 2.16 | 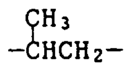 | 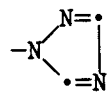 | |
| 2.17 | 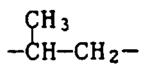 | 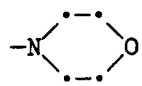 | |

Tabelle 7

Verbindungen der Formel

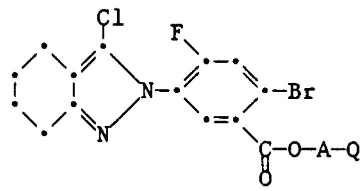

| Verb. Nr. | A | Q | physikalische Daten |
|---|---|---|---|
| 7.01 | $-CH_2CH_2-$ | $-SCH_3$ | |
| 7.02 | $-\overset{CH_3}{C}HCH_2-$ | $-SCH_3$ | |
| 7.03 | $-\overset{CH_3}{C}HCH_2-$ | $-SC_2H_5$ | |
| 7.04 | $-\overset{CH_3}{C}HCH_2-$ | $-SC_3H_7(i)$ | |
| 7.05 | $-\overset{CH_3}{C}HCH_2-$ | $-SC_4H_9$ | |
| 7.06 | $-\overset{CH_3}{C}HCH_2-$ | $-SC_4H_9(s)$ | |
| 7.07 | $-\overset{CH_3}{C}HCH_2-$ | $-SC_4H_9(t)$ | |
| 7.08 | $-\overset{CH_3}{C}HCH_2-$ | $-SC_8H_{17}$ | |
| 7.09 | $-\overset{CH_3}{C}HCH_2-$ | $-SCH_2-$ 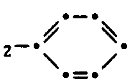 | |
| 7.10 | $-\overset{CH_3}{C}HCH_2-$ | 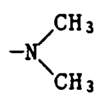 | |
| 7.11 | $-CH_2CH_2-$ | 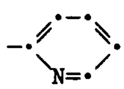 | |
| 7.12 | $-CH_2CH_2-$ | 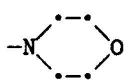 | |
| 7.13 | $-CH_2CH_2-$ | 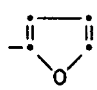 | |
| 7.14 | $-\overset{CH_3}{C}HCH_2-$ | 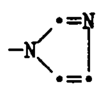 | |

Tabelle 7 (Fortsetzung)

| Verb. Nr. | A | Q | physikalische Daten |
|---|---|---|---|
| 7.15 | 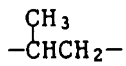 | 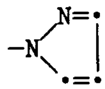 | |
| 7.16 | 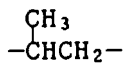 | 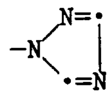 | |
| 7.17 | 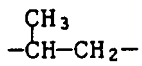 | 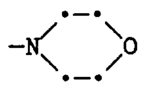 | |

Tabelle 8

Verbindungen der Formel

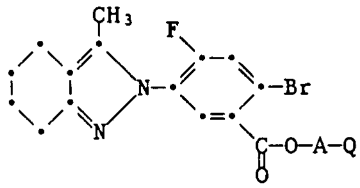

| Verb. Nr. | A | Q | physikalische Daten |
|---|---|---|---|
| 8.01 | $-CH_2CH_2-$ | $-SCH_3$ | |
| 8.02 | $-\overset{CH_3}{\overset{\vert}{C}}HCH_2-$ | $-SCH_3$ | |
| 8.03 | $-\overset{CH_3}{\overset{\vert}{C}}HCH_2-$ | $-SC_2H_5$ | |
| 8.04 | $-\overset{CH_3}{\overset{\vert}{C}}HCH_2-$ | $-SC_3H_7(i)$ | |
| 8.05 | $-\overset{CH_3}{\overset{\vert}{C}}HCH_2-$ | $-SC_4H_9$ | |
| 8.06 | $-\overset{CH_3}{\overset{\vert}{C}}HCH_2-$ | $-SC_4H_9(s)$ | |
| 8.07 | $-\overset{CH_3}{\overset{\vert}{C}}HCH_2-$ | $-SC_4H_9(t)$ | |
| 8.08 | $-\overset{CH_3}{\overset{\vert}{C}}HCH_2-$ | $-SC_8H_{17}$ | |
| 8.09 | $-\overset{CH_3}{\overset{\vert}{C}}HCH_2-$ | $-SCH_2-$ 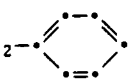 | |
| 8.10 | $-\overset{CH_3}{\overset{\vert}{C}}HCH_2-$ | 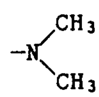 | |
| 8.11 | $-CH_2CH_2-$ | 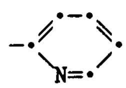 | |
| 8.12 | $-CH_2CH_2-$ | 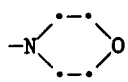 | |
| 8.13 | $-CH_2CH_2-$ | 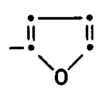 | |
| 8.14 | $-\overset{CH_3}{\overset{\vert}{C}}HCH_2-$ | 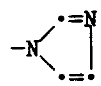 | |

Tabelle 8 (Fortsetzung)

| Verb. Nr. | A | Q | physikalische Daten |
|---|---|---|---|
| 8.15 | 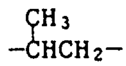 | 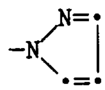 | |
| 8.16 | 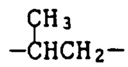 | 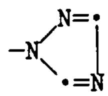 | |
| 8.17 | 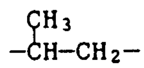 | 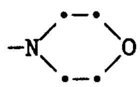 | |

F. Formulierungsbeispiele

Beispiel F 1.: Formulierungsbeispiele für Wirkstoffe der Formel I (% = Gewichtsprozent)

| a) Emulsionskonzentrate | | | |
|---|---|---|---|
| | a) | b) | c) |
| Wirkstoff gemäss Tabellen 1, 2, 7 und 8 | 20% | 40% | 50 % |
| Ca-Dodecylbenzolsulfonat | 5 % | 8 % | 5,8 % |
| Ricinusöl-polyethylenglykolether (36 Mol EO) | 5 % | - | - |
| Tributylphenoyl-polyethylenglykolether (30 Mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | | | |
|---|---|---|---|
| | a) | b) | c) |
| Wirkstoff gemäss Tabellen 1, 2, 7 und 8 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | | |
|---|---|---|
| | a) | b) |
| Wirkstoff gemäss Tabellen 1, 2, 7 und 8 | 5 % | 10 % |
| Kaolin | 94 % | - |
| Hochdisperse Kieselsäure | 1 % | |
| Attapulgit | - | 90 % |

Der Wirkstoff wird gelöst, auf den Träger aufgesprüht und das Lösungsmittel anschliessend im Vakuum abgedampft.

| d) Stäubemittel | | | |
|---|---|---|---|
| | a) | b) | c) |
| Wirkstoff gemäss Tabellen 1, 2, 7 und 8 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

| e) Spritzpulver | | |
|---|---|---|
| | a) | b) |
| Wirkstoff gemäss Tabellen 1, 2, 7 und 8 | 20 % | 60 % |
| Na-Ligninsulfonat | 5 % | 5 % |
| Na-Laurylsulfat | 3 % | 6 % |
| Octylphenolpolyethylenglykolether (7-8 Mol EO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Tabellen 1, 2, 7 und 8 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| g) Umhüllungs-Granulat | |
|---|---|
| Wirkstoff gemäss Tabellen 1, 2, 7 und 8 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| h) Suspensions-Konzentrat | |
|---|---|
| Wirkstoff gemäss Tabellen 1, 2, 7 und 8 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

B.1.: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe behandelt. Es wird eine Konzentrationen von 4 kg Wirksubstanz/Hektar angewendet. Die Saatschalen werden im Gewächshaus bei 22-25 °C und 50-70 % relativer Luftfeuchtigkeit gehalten und der Versuch nach 3 Wochen ausgewertet.

Die herbizide Wirkung wird anhand eines linearen neunstufigen Bonitierungsschemas bewertet, in welchem 1 für totale Schädigung (100 % Herbizidwirkung) und 9 für keine Wirkung (Versuchspflanze wächst wie die unbehandelte Referenz) steht.

Einzelergebnisse sind in Tabelle 13 zusammengestellt.

Tabelle 13

| pre emergente Herbizidwirkung bei 250 g/ha-Aufwandmenge. | | | | |
|---|---|---|---|---|
| Verb. No. | Avena | Sinapis | Setaria | Stellaria |
| 1.03 | 2 | 1 | 1 | 1 |
| 1.04 | 3 | 1 | 1 | 1 |
| 1.05 | 2 | 1 | 1 | 1 |
| 1.06 | - | 1 | 1 | 1 |
| 1.08 | - | - | 1 | 1 |
| 1.17 | - | 1 | 1 | 1 |
| 1.18 | - | - | 1 | 1 |
| 1.20 | - | - | 1 | 1 |

B.2.: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24 °-26 °C und 45-60 % relativer Luftfeuchtigkeit gehalten. 15 Tage nach der Behandlung wird der Versuch ausgewertet.

Die herbizide Wirkung wird analog zu Beispiel B1 bonitiert.

Einzelergebnisse sind in Tabelle 14 zusammengestellt.

Tabelle 14

| post emergente Herbizidwirkung bei 4 kg/ha Aufwandmenge. | | | | | | | |
|---|---|---|---|---|---|---|---|
| Verb. No. | Avena | Setaria | Lolium | Solanum | Sinapis | Stellaria | Phaseolus |
| 1.03 | 2 | 2 | 1 | 1 | 1 | 1 | 1 |
| 1.04 | 1 | 1 | 1 | 1 | 1 | 1 | 1 |
| 1.05 | 1 | 1 | 1 | 1 | 1 | 1 | 1 |
| 1.06 | 1 | 1 | 1 | 1 | 1 | 1 | 1 |
| 1.08 | 2 | 1 | 1 | 1 | 1 | 1 | 1 |
| 1.17 | 2 | 2 | 1 | 1 | 1 | 1 | 1 |
| 1.18 | 1 | 1 | 1 | 1 | 1 | 1 | 1 |
| 1.20 | - | 3 | 1 | 1 | 1 | 1 | - |
| 4.10 | - | - | 2 | 1 | 1 | 2 | 1 |

B.3.: Herbizidwirkung für Wasserunkräuter (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Dispersion der Prüfsubstanzen durch eine Spritzung auf die Gefässe mit einer Aufwandmenge von 250 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt.

Die Bonitierung wird gemäss der in Beispiel B1 angegebenen Skala durchgeführt.

Einzelergebnisse sind in Tabelle 15 zusammengestellt.

Tabelle 15

| Verb. Nr. | Echinochloa | Monocharia |
|---|---|---|
| 1.03 | 1 | 1 |
| 1.04 | 1 | 1 |
| 1.05 | 1 | 1 |
| 1.06 | 1 | 1 |
| 1.08 | 1 | 1 |
| 1.17 | 1 | 1 |
| 1.20 | 1 | 1 |
| 4.10 | 3 | 1 |

B.4.: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe behandelt. Es werden unterschiedliche Aufwandmengen Wirksubstanz/Hektar getestet. Die Saatschalen werden im Gewächshaus bei 22-25°C und 50-70 % relativer Luftfeuchtigkeit gehalten und der Versuch nach 3 Wochen ausgewertet.

Die Herbizidwirkung wird dabei in einem neunstufigen Boniturschema (wie im Beispiel B1 beschrieben) im Vergleich zur unbehandelten Kontrollgruppe ausgewertet.

Boniturnoten von 1 bis 4 (insbesondere von 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin. Die Testresultate sind in Tabelle 16 zusammengefasst.

Tabelle 16

| Verb. No. | 1.03 | | | 1.17 | | |
|---|---|---|---|---|---|---|
| Aufwandmenge [g/ha] | 1000 | 500 | 250 | 1000 | 500 | 250 |
| Gerste | 6 | 8 | 9 | 9 | 9 | 9 |
| Weizen | 6 | 8 | 9 | 9 | 9 | 9 |
| Mais | 7 | 8 | 9 | 7 | 8 | 9 |
| Sorghum hybr. | 8 | 9 | 9 | 6 | 7 | 9 |
| Reis (trocken) | 7 | 8 | 9 | 6 | 8 | 9 |
| Rottboellia ex. | 1 | 1 | 1 | 6 | 8 | 9 |
| Soja | 9 | 9 | 9 | 9 | 9 | 9 |
| Baumwolle | 7 | 9 | 9 | 9 | 9 | 9 |
| Sonnenblume | 8 | 9 | 9 | 9 | 9 | 9 |
| Abutilon | 1 | 1 | 1 | 1 | 1 | 4 |
| Sida spinosa | 1 | 2 | 3 | 3 | 3 | 4 |
| Amaranthus ret. | 1 | 1 | 1 | 1 | 5 | 7 |
| Chenopodium Sp. | 1 | 1 | 1 | 1 | 1 | 1 |
| Solanum nigrum | 1 | 1 | 1 | 1 | 1 | 1 |
| Chrysanthe. Leuc. | 1 | 1 | 1 | 1 | 1 | 2 |
| Viola tricolor | 1 | 1 | 1 | 1 | 1 | 1 |
| Veronica Sp. | 1 | 1 | 1 | 1 | 1 | 1 |

B.5.: Post-emergente Herbizid-Wirkung

Eine Anzahl Unkräuter, sowohl monokotyle wie dikotyle, werden nach dem Auflaufen (im 4- bis 6-Blattstadium) mit einer wässrigen Wirkstoffdispersion in einer Dosierung von 250 g bis 1 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach dem in Beispiel B1 beschriebenen Boniturschema ausgewertet.

Die Testresultate für Verbindungen 1.03 und 1.17 sind in Tabelle 17 zusammengestellt.

Tabelle 17

| Verb. No. | 1.03 | | | | | | 1.17 | | | | | |
|---|---|---|---|---|---|---|---|---|---|---|---|---|
| Aufwandmenge [g/ha] | 1000 | 500 | 250 | 125 | 60 | 30 | 1000 | 500 | 250 | 125 | 60 | 30 |
| Gerste | 4 | 5 | 6 | 7 | 8 | 8 | 5 | 6 | 7 | 8 | 8 | 8 |
| Weizen | 4 | 5 | 6 | 7 | 8 | 8 | 6 | 7 | 8 | 8 | 9 | 9 |
| Mais | 3 | 4 | 6 | 7 | 8 | 9 | 4 | 4 | 5 | 7 | 8 | 9 |
| Sorghum hybr. | 4 | 5 | 6 | 7 | 8 | 8 | 7 | 7 | 8 | 8 | 9 | 9 |
| Abutilon | 1 | 1 | 1 | 1 | 1 | 1 | 1 | 1 | 1 | 1 | 1 | 1 |
| Sida spinosa | 1 | 1 | 1 | 1 | 4 | 5 | 1 | 1 | 2 | 3 | 4 | 6 |
| Xanthium Sp. | 1 | 1 | 1 | 1 | 1 | 2 | 1 | 1 | 1 | 1 | 4 | 4 |
| Amaranthus ret. | 1 | 1 | 1 | 1 | 1 | 4 | 1 | 1 | 1 | 4 | 4 | 4 |
| Chenopodium Sp. | 1 | 1 | 1 | 1 | 1 | 1 | 1 | 1 | 1 | 1 | 1 | 2 |
| Solanum nigrum | 1 | 1 | 1 | 1 | 1 | 1 | 1 | 1 | 1 | 1 | 1 | 1 |
| Ipomoea | 1 | 1 | 1 | 1 | 1 | 4 | 1 | 1 | 1 | 1 | 4 | 4 |
| Sinapis | 1 | 1 | 1 | 1 | 1 | 4 | 1 | 2 | 3 | 4 | 5 | 6 |
| Chrysanthe. Leuc. | 1 | 1 | 1 | 1 | 4 | 5 | 1 | 1 | 1 | 2 | 4 | 6 |
| Viola tricolor | 1 | 1 | 1 | 1 | 1 | 1 | 1 | 1 | 1 | 1 | 1 | 1 |
| Veronica Sp. | 1 | 1 | 1 | 1 | 1 | 2 | 1 | 1 | 1 | 1 | 4 | 6 |

## Patentansprüche

**1.** Verbindungen der Formel I

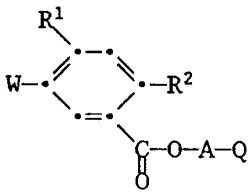

(I),

worin

W

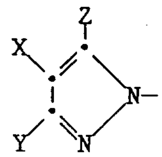

;

| | |
|---|---|
| $R^1$ | Wasserstoff; oder Halogen; |
| $R^2$ | Halogen; |
| A | eine geradkettige oder verzweigte $C_1$-$C_4$-Alkylenkette; |
| n | 0; oder 1; |
| Q | -$S(O)_m$-$R^3$; Di-$C_1$-$C_4$-Alkylamino; einen über Kohlenstoff gebundenen 6-gliedrigen Heterocyclus, welcher 1 bis 3 Stickstoffatome enthält, wobei dieser Heterocyclus seiner seits noch bis zu zweifach durch Halogen, $C_1$-$C_4$-Alkyl, oder $C_1$-$C_3$-Halogenalkyl substituiert sein kann; |
| $R^3$ | $C_1$-$C_{10}$-Alkyl; |
| m | 0; 1; oder 2; |
| X | Wasserstoff; Halogen; oder $C_1$-$C_4$-Alkyl; |
| Y | $C_1$-$C_4$-Alkyl; oder |
| X und Y | gemeinsam eine gegebenenfalls bis zu zweifach durch Methyl substituierte -$(CH_2)_q$-Kette; |
| q | 3; oder 4; und |
| Z | Halogen; Methyl; $C_1$-$C_6$-Alkoxy; oder $C_1$-$C_6$-Alkylthio; |

bedeutet.

**2.** Verbindungen der Formel I gemäss Anspruch 1 worin

| | |
|---|---|
| W | wie zuvor definiert ist und |
| $R^1$ | Wasserstoff; Fluor; oder Chlor; |
| $R^2$ | Fluor; Chlor; oder Brom; |
| A | eine geradkettige oder verzweigte $C_2$-$C_4$-Alkylenkette; |
| n | 0; oder 1; |
| Q | $C_1$-$C_{10}$-Alkylthio; Di-$C_1$-$C_4$-Alkylamino; gegebenenfalls bis zu zweifach durch $C_1$-$C_4$-Alkyl substituiertes Pyridinyl; |
| X | Wasserstoff; Chlor; oder Brom; |
| Y | $C_1$-$C_4$-Alkyl; oder |
| X und Y | gemeinsam eine -$(CH_2)_q$-Kette; |
| q | 3; oder 4; und |
| Z | Chlor; Brom; Methyl; $C_1$-$C_6$-Alkoxy; oder $C_1$-$C_6$-Alkylthio; |

bedeutet.

**3.** Verbindungen der Formel I gemäss Anspruch 2 worin

| | |
|---|---|
| W | wie zuvor definiert ist und |
| $R^1$ | Wasserstoff; Fluor; oder Chlor; |

| | |
|---|---|
| $R^2$ | Chlor; oder Brom; |
| A | Ethano; 1-Methylethano; 2-Methylethano; oder Propano; |
| n | 0; oder 1; |
| Q | $C_1$-$C_5$-Alkylthio; Pyridin-2-yl; |
| X und Y | gemeinsam eine $-(CH_2)_4-$ Kette; und |
| Z | Chlor; oder Methyl; |

bedeutet.

**4.** Verbindungen der Formel I, gemäss einem der Ansprüche 1 bis 3, worin
W

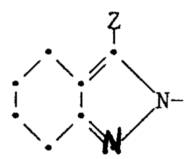

und
Z Chlor; oder Methyl;
bedeutet.

**5.** Verbindungen der Formel I, gemäss einem oder mehreren der Ansprüche 1 bis 4 worin A $-CH_2CH_2-$,

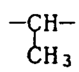 oder 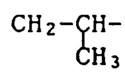

bedeutet.

**6.** Verbindungen der Formel I, gemäss einem oder mehreren der Ansprüche 1 bis 5, worin Q $C_1$-$C_6$-Alkylthio bedeutet.

**7.** Verbindungen der Formel I, gemäss einem oder mehreren der Ansprüche 1 bis 5, worin Q di-$C_1$-$C_4$-Alkylamino bedeutet.

**8.** Verbindungen der Formel I, gemäss einem oder mehreren der Ansprüche 1 bis 7, worin $R^1$ Fluor; und $R^2$ Chlor; oder Brom; bedeutet.

**9.** Verbindungen der Formel I, gemäss einem der Ansprüche 1 bis 3, worin
W

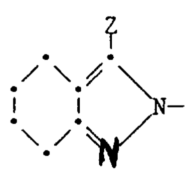

und
Z Chlor; oder Methyl;
A $-CH_2-CH_2-$; oder

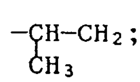

und
Q $C_1$-$C_6$-Alkylthio bedeutet.

**10.** Verbindungen der Formel I, gemäss Anspruch 1, worin
W

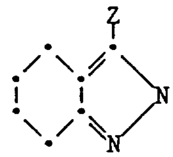

Z Chlor; oder Methyl;
A $-CH_2-CH_2-$; oder

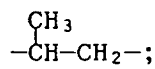

Q $C_1$-$C_6$-Alkylthio;
$R^1$ Fluor; und
$R^2$ Chlor; oder Brom;
bedeutet.

**11.** 3-Chlor-2-{4-chlor-2-fluor-5-[1-methyl-2-(1-methylethylthio)-ethoxy-carbonyl]-phenyl}-4,5,6,7-tetrahydro-2H-indazol,
3-Chlor-2-[4-chlor-2-fluor-5-(1-dimethylamino-1-methylethoxycarbonyl)-phenyl]-4,5,6,7-tetrahydro-1H-indazol oder
3-Chlor-2-[4-chlor-2-fluor-5-(2-ethylthio-1-methylethoxycarbonyl)-phenyl]-4,5,6,7-tetrahydro-2H-indazol
als Verbindungen der Formel I gemäss Anspruch 1.

**12.** Verfahren zur Herstellung von Verbindungen der Formel I gemäss einem oder mehreren der Ansprüche 1 bis 11 gekennzeichnet durch die Veresterung eines Carbonsäurederivats der Formel II, worin $R^1$, $R^2$ und W wie zuvor definiert sind und $R^5$ OH, Chlor oder Brom bedeutet, mit einem Alkohol der Formel III, worin A und Q wie zuvor definiert sind.

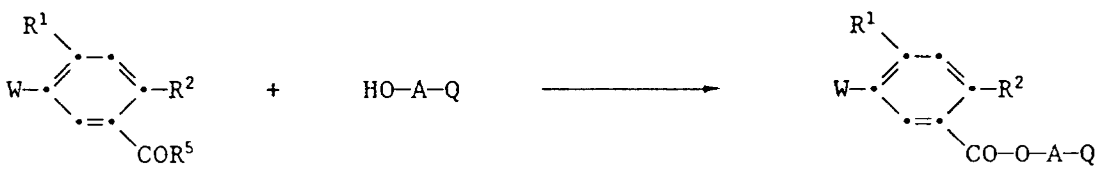

II III I .

**13.** Verfahren zur Herstellung von Verbindungen der Formel I'', worin die Reste W, $R^1$, $R^2$, $R^3$ und A wie in einem der Ansprüche 1 bis 11 definiert sind und Q für $-S(O)_mR^3$ und m für 1 oder 2 steht, gekennzeichnet durch die Oxidation eines Thioethers der Formel I', worin W, $R^1$, $R^2$, $R^3$ und A wie zuvor definiert sind und m für 0 steht

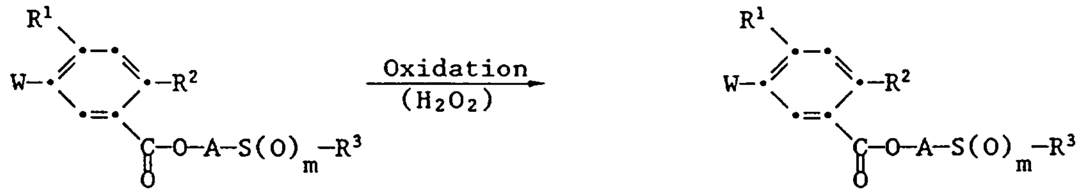


I' (m = 0) I" (m = 1,2).

**14.** Herbizides Mittel enthaltend als Wirkstoff mindestens eine Verbindung der Formel I gemäss einem oder mehreren der Ansprüche 1 bis 11 neben weiteren Hilfs- und oder Trägerstoffen.

**15.** Verfahren zur Bekämpfung unerwünschten Pflanzenwuchses, dadurch gekennzeichnet, dass man die unerwünschte Pflanze oder deren Lebensraum mit einer Verbindung der Formel I gemäss einem oder mehreren der Ansprüche 1 bis 11 behandelt.

**16.** Verfahren gemäss Anspruch 15 zur pre- oder postemergenten Bekämpfung unerwünschten Pflanzenwuchses in Nutzpflanzenkulturen.

**17.** Verfahren gemäss Anspruch 16 in Kulturen von Gerste, Weizen, Mais, Reis, Sorghum, Soja, Baumwolle oder Sonnenblumen.

**18.** Saatgut welches mit einer herbizid wirksamen Menge einer Verbindung der Formel I gemäss einem oder mehreren der Ansprüche 1 bis 11 oder einem Mittel gemäss Anspruch 14 behandelt worden ist.

## Claims

**1.** A compound of formula I

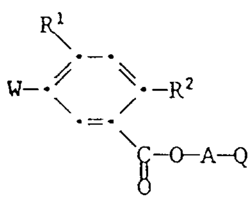

(I),

wherein

W is

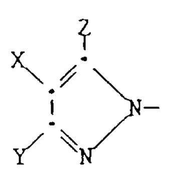

;

| | |
|---|---|
| $R^1$ | is hydrogen or halogen; |
| $R^2$ | is halogen; |
| A | is a straight-chain or branched $C_1$-$C_4$alkylene chain; |
| n | is zero or 1; |
| Q | is -$S(O)_m$-$R^3$; di-$C_1$-$C_4$alkylamino; a 6-membered heterocycle which is bonded via a carbon atom and which contains 1 to 3 nitrogen atoms, which heterocycle can in turn be mono- or di-substituted by halogen, $C_1$-$C_4$alkyl or by $C_1$-$C_3$haloalkyl; |

$R^3$ is $C_1$-$C_{10}$alkyl;
m is zero, 1 or 2;
X is hydrogen, halogen or $C_1$-$C_4$alkyl;
Y is $C_1$-$C_4$alkyl; or
X and Y together form a -$(CH_2)_q$- chain, which is optionally mono- or di-substituted by methyl;
q is three or four; and
Z is halogen, methyl, $C_1$-$C_6$alkoxy or $C_1$-$C_6$alkylthio.

**2.** A compound of formula I according to claim 1, wherein
W is as defined above;
$R^1$ is hydrogen, fluorine or chlorine;
$R^2$ is fluorine, chlorine or bromine;
A is a straight-chain or branched $C_2$-$C_4$alkylene chain;
n is zero or 1;
Q is $C_1$-$C_{10}$alkylthio; di-$C_1$-$C_4$alkylamino; or pyridinyl which is optionally mono- or di-substituted by $C_1$-$C_4$alkyl;
X is hydrogen, chlorine or bromine;
y is $C_1$-$C_4$alkyl; or
X and Y together form a -$(CH_2)_q$- chain;
q is 3 or 4; and
Z is chlorine, bromine, methyl, $C_1$-$C_6$alkoxy or $C_1$-$C_6$alkylthio.

**3.** A compound of formula I, according to claim 2, wherein
W is as defined above;
$R^1$ is hydrogen, fluorine or chlorine;
$R^2$ is chlorine or bromine;
A is ethano, 1-methylethano, 2-methylethano or propano;
n is zero or 1;
Q is $C_1$-$C_5$alkylthio or pyridin-2-yl,
X and Y together form a -$(CH_2)_4$- chain; and
Z is chlorine or methyl.

**4.** A compound of formula I, according to any one of claims 1 to 3, wherein
W is

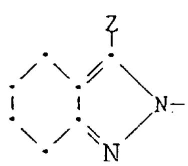

wherein Z is chlorine or methyl.

**5.** A compound of formula I, according to one or more of claims 1 to 4, wherein A is -$CH_2$-$CH_2$-,

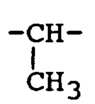 or 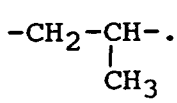

**6.** A compound of formula I, according to one or more of claims 1 to 5, wherein Q is $C_1$-$C_6$alkylthio.

**7.** A compound of formula I, according to one or more of claims 1 to 5, wherein Q is di-$C_1$-$C_4$alkylamino.

**8.** A compound of formula I, according to one or more of claims 1 to 7, wherein $R^1$ is fluorine and $R^2$ is chlorine or bromine.

**9.** A compound of formula I, according to any one of claims 1 to 3, wherein
W is

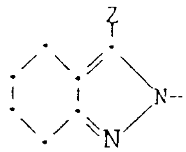

and
Z is chlorine or methyl;
A is $-CH_2-CH_2-$; or

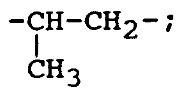

and
Q is $C_1-C_6$ alkylthio.

**10.** A compound of formula I, according to claim 1, wherein
W is

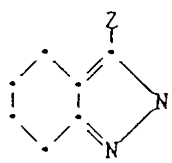

Z is chlorine; or methyl;
A is $-CH_2-CH_2-$; or

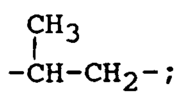

Q is $C_1-C_6$ alkylthio;
$R^1$ is fluorine; and
$R^2$ is chlorine; or bromine.

**11.** 3-Chloro-2-{4chloro-2-fluoro-5-[1-methyl-2-(1-methylethylthio)-ethoxycarbonyl]-phenyl}-4,5,6,7-tetrahydro-2H-indazole,
3-chloro-2-[4-chloro-2-fluoro-5-(1-dimethylamino-1-methylethoxycarbonyl)-phenyl]-4,5,6,7-tetrahydro-1H-indazole or
3-chloro-2-[4-chloro-2-fluoro-5-(2-ethylthio-1-methyl-ethoxycarbonyl)-phenyl]-4,5,6,7-tetrahydro-2H-indazole as a compound of formula I according to claim 1.

**12.** A process for the production of a compound of formula I according to one or more of claims 1 to 11, which comprises esterifying a carboxylic acid derivative of formula II, wherein $R^1$, $R^2$ and W are as defined above and $R^5$ is hydroxy, chlorine or bromine, with an alcohol of formula III, wherein A and Q are as defined above

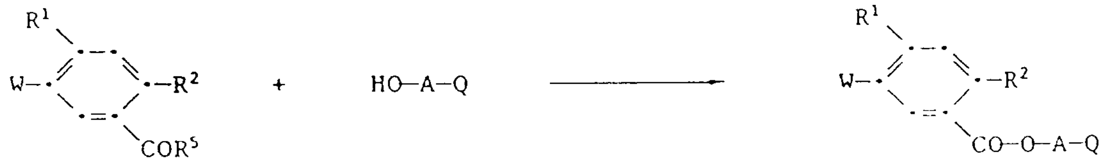

II III I .

13. A process for the production of a compound of formula I", wherein the radicals W, $R^1$, $R^2$, $R^3$ and A are as defined in any one of claims 1 to 11 and Q is $-S(O)_m-R^3$ and m is 1 or 2, which comprises the oxidation of a thioether of formula I', wherein W, $R^1$, $R^2$, $R^3$ and A are as defined above and m is 0.

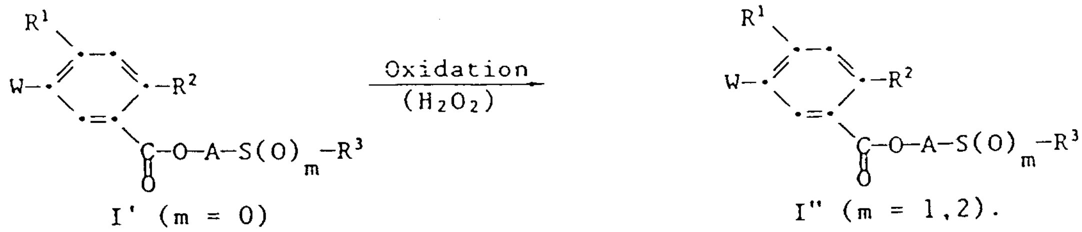


I' (m = 0) I" (m = 1,2).

14. A herbicidal composition, which comprises besides further auxiliaries and/or carrier materials, as active ingredient, at least one compound of formula I according to one or more of claims 1 to 11.

15. A method for controlling undesired plant growth, which comprises treating the undesired plant or its living area with a compound of formula I according to one or more of claims 1 to 11.

16. A method according to claim 15 for the pre- or post-emergence control of undesired plant growth in cultures of crop plants.

17. A method according to claim 16 in crops of barley, wheat, maize, rice, sorghum, soya, cotton or sunflowers.

18. Seeds which have been treated with a herbicidally effective amount of a compound of formula I according to one or more of claims 1 to 11 or of a composition according to claim 14.

**Revendications**

1. Composé de formule I

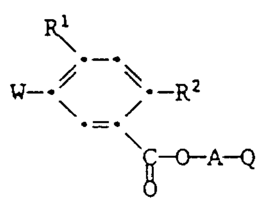

(I),

où

W représente

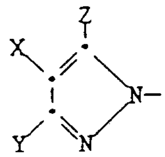 ;

| | |
|---|---|
| $R^1$ | l'hydrogène; ou un halogène; |
| $R^2$ | un halogène; |
| A | une chaîne alkylène en $C_1$-$C_4$ linéaire ou ramifiée; |
| n | est égal à 0, ou 1; |
| Q | représente -$S(O)_m$-$R^3$; un dialkyle en $C_1$-$C_4$ amino; un hétérocycle à 6 chaînons contenant 1 à 3 atomes d'azote, lié par le carbone, cet hétérocycle pouvant être lui-même encore jusqu'à deux fois substitué par un halogène, un alkyle en $C_1$-$C_4$ ou un halogénoalkyle en $C_1$-$C_3$; |
| $R^3$ | représente un alkyle en $C_1$-$C_{10}$; |
| m | est égal à 0; 1; ou 2; |
| X | représente l'hydrogène; un halogène; ou un alkyle en $C_1$-$C_4$; |
| Y | un alkyle en $C_1$-$C_4$; ou |
| X et Y | représentent ensemble une chaîne -$(CH_2)_q$-éventuellement jusqu'à deux fois substituée par le méthyle; |
| q | est égal à 3; ou 4; et |
| Z | représente un halogène; le méthyle; un alcoxy en $C_1$-$C_6$; ou un alkyle en $C_1$-$C_6$ thio. |

**2.** Composés de formule I selon la revendication 1, où

| | |
|---|---|
| W | est défini comme précédemment et |
| $R^1$ | représente l'hydrogène; le fluor; ou le chlore; |
| $R^2$ | le fluor; le chlore; ou le brome; |
| A | une chaîne alkylène en $C_2$-$C_4$ linéaire ou ramifiée; |
| n | est égal à 0; ou 1; |
| Q | représente un alkyle en $C_1$-$C_{10}$ thio; un dialkyle en $C_1$-$C_4$ amino; un pyridinyle éventuellement jusqu'à deux fois substitué par un alkyle en $C_1$-$C_4$; |
| X | l'hydrogène; le chlore; ou le brome; |
| Y | un alkyle en $C_1$-$C_4$; ou |
| Y | un alkyle en $C_1$-$C_4$; ou |
| X et Y | représentent ensemble une chaîne -$(CH_2)_q$- |
| q | est égal à 3; ou 4; et |
| Z | représente le chlore; le brome; le méthyle; un alcoxy en $C_1$-$C_6$; ou un alkyle en $C_1$-$C_6$ thio; |

**3.** Composés de formule I selon la revendication 2, ou

| | |
|---|---|
| W | est défini comme précédemment et |
| $R^1$ | représente l'hydrogène; le fluor; ou le chlore; |
| $R^2$ | le chlore; ou le brome; |
| A | l'éthano; le l-méthyléthano; le 2-méthyléthano; ou le propano; |
| n | est égal à 0; ou 1; |
| Q | représente un alkyle en $C_1$-$C_5$ thio; ou le pyridin-2-yle; |
| X et Y | représentent ensemble une chaîne -$(CH_2)_4$-; et |
| Z | représente le chlore; ou le méthyle. |

**4.** Composés de formule I selon l'une des revendications 1 à 3, où

W représente

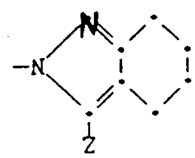

et

Z le chlore; ou le méthyle.

**5.** Composés de formule I selon l'une ou plusieurs des revendications 1 à 4, où A représente $-CH_2-CH_2-$;

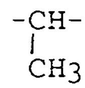 ou 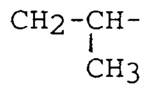

**6.** Composés de formule I selon l'une ou plusieurs des revendications 1 à 5, où Q représente un alkyle en $C_1$-$C_6$ thio.

**7.** Composés de formule I selon l'une ou plusieurs des revendications 1 à 5, où Q représente un dialkyle en $C_1$-$C_4$ amino.

**8.** Composés de formule I selon l'une ou plusieurs des revendications 1 à 7, om $R^1$ représente le fluor; et $R^2$ le chlore; ou le brome.

**9.** Composés de formule I selon l'une des revendications 1 à 3, où

W représente

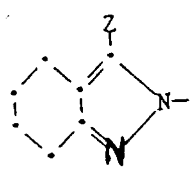

et

Z le chlore; ou le méthyle;

A $-CH_2-CH_2-$; ou

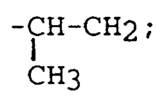

et-

Q un alkyle en $C_1$-$C_6$ thio.

**10.** Composés de formule I selon la revendication 1, où

W représente

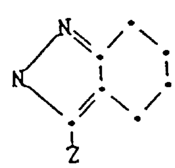

Z le chlore; ou le méthyle;

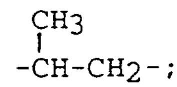

A $-CH_2-CH_2-$; ou
Q un alkyle en $C_1$-$C_6$ thio;
$R^1$ le fluoor; et
$R^2$ le chlore; ou le brome.

**11.** Le 3-chloro-2-{4-chloro-2-fluoro-5-[1-méthyl-2-(1-méthyléthylthio)-éthoxycarbonyl]-phényl}-4,5,6,7-tétrahydro-2H-indazole, le 3-chloro-2-[4-chloro-2-fluoro-5-(1-diméthylamino-1-méthyléthoxy-carbonyl)-phényl]-4,5,6,7,tétrahydro-1H-indazole et le 3-chlor o-2-[4-chloro-2-fluoro-5-(2-éthylthio-1-méthyléthoxy-carbonyl)-phényl]-4,5,6,7-tétrahydro-2H-indazole comme composés de formule I selon la revendication 1.

**12.** Procédé pour la préparation des composés de formule I selon l'une ou plusieurs des revendications 1 à 11, caractérisé par l'estérification d'un dérivé d'acide carboxylique de formule II où $R^1$, $R^2$ et W sont définis comme précédemment et $R^5$ représente OH, le chlore ou le brome, avec un alcool de formule III où A et Q sont définis comme précédemment:

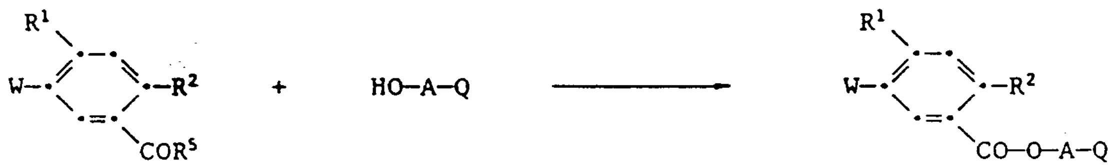


II III I .

**13.** Procédé pour la préparation des composés de formule I" où les restes W, $R^1$, $R^2$, $R^3$ et A sont définis comme dans l'une des revendications 1 à 11 et Q représente $-S(O)_m-R^3$ et m est égal à 1 ou 2, caractérisé par l'oxydation d'un thioéther de formule I' où W, $R^1$, $R^2$, $R^3$ et A sont définis comme précédemment et m est égal à 0.

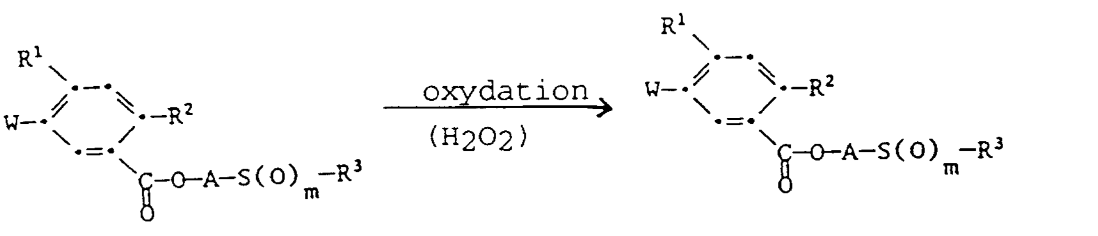


I' (m = 0) I'' (m = 1 ou 2)

**14.** Composition herbicide contenant comme substance active au moins un composé de formule I selon l'une ou plusieurs des revendications 1 à 11, à côté d'autres adjuvants et/ou supports.

**15.** Procédé pour lutter contre la croissance des plantes indésirables, caractérisé en ce que l'on traite les plantes indésirables où l'endroit où elles se trouvent avec un composé de formule I selon l'une ou plusieurs des revendications 1 à 11.

**16.** Procédé selon la revendication 15 pour lutter contre la croissance des plantes indésirables en pré- ou en post-émergence dans la culture de plantes utiles.

**17.** Procédé selon la revendication 16 pour lutter contre la croissance des plantes indésirables en pré- ou en post-émergence dans les cultures d'orge, de blé, de maïs, de riz, de sorgho, de soja, de coton ou de tournesol.

**18.** Semences ayant été traitées avec une quantité efficace comme herbicide d'un composé de formule I selon l'une ou plusieurs des revendications 1 à 11 ou avec une composition selon la revendication 14.